# FASCICULE DE BREVET EUROPEEN

(11) **EP 1 231 892 B1**
(45) Date de publication et mention de la délivrance du brevet: **08.09.2004**
(21) Numéro de dépôt: 00981452.6
(22) Date de dépôt: 22.11.2000
(51) Int. Cl.: A61K 7/48, A61K 35/78

(54) **COMPOSITION DE SOINS COMPRENANT ENTRE AUTRES DE L'AMIDON DE RIZ, DE L'HUILE DE COCO ET DU BEURRE DE KARITE**
PFLEGEMITTEL ENTHALTEND U.A. REISSTÄRKE, KOKOSÖL UND KARITE-BUTTER
COSMETIC SKIN CARE COMPOSITION COMPRISING INTER ALIA RICE STARCH, COCONUT OIL AND KARITE BUTTER

(30) Priorité: 26.11.1999 FR 9914916
(43) Date de publication de la demande: 21.08.2002
(73) Titulaire: Ruel, Arnaud, 83200 Toulon (FR)
(72) Inventeur: Ruel, Arnaud, 83200 Toulon (FR)
(74) Mandataire: Santarelli
(86) Numéro de dépôt international: PCT/FR2000/003239
(87) Numéro de publication internationale: WO 2001/037792

(56) Documents cités:
- EP-A- 0 332 501
- EP-A- 0 897 718
- FR-A- 2 520 615
- FR-A- 2 597 337
- FR-A- 2 697 433

## Description

La présente invention concerne une nouvelle composition dermo-cosmétologique, son procédé de préparation et ses applications.

Depuis très longtemps, l'homme a recherché à améliorer son apparence, notamment plus il avance en âge. Cette tendance ne fait que s'accentuer dans le temps. C'est pourquoi on recherche toujours des produits cosmétiques à ingérer ou à appliquer par voie externe, pour améliorer l'apparence ou retarder le vieillissement de la peau.

Après de longues recherches mettant en jeu de très nombreux constituants différents, le demandeur a mis au point une nouvelle composition dotée de nombreuses qualités.

Cette composition se fonde essentiellement sur six constituants, parmi lesquels deux de ceux-ci jouent un rôle plus particulier. Il s'agit de l'extrait de coco et de l'amidon de riz qui, pour des raisons que la demanderesse n'a pas réussi à élucider, semblent se comporter comme des agents catalyseurs pour les autres constituants de la composition.

C'est pourquoi la présente demande a pour objet une composition dermo-cosmétologique caractérisée en ce qu'elle comprend de l'amidon de riz, du coco sous forme de poudre de pulpe, de beurre ou d'huile, du beurre de karité, de l'huile de bourrache, de l'huile d'avocat et de l'huile de jojoba.

Le document brevet EP-A-0 332 501 décrit théoriquement l'utilisation d' amidon de riz, de beurre de karité et d' huiles de bourrache, de jojoba et d' avocat dans composition dermocosmétique de la colonne 4, ligne 13 à colonne 5, ligne 58.

Dans des conditions préférentielles de mise en oeuvre de l'invention, la composition ci-dessus renferme en outre de l'huile de noix d'argan qui est le fruit de l'arganier.

Dans d'autres conditions préférentielles de mise en oeuvre de l'invention, les compositions ci-dessus renferment en outre de l'huile de rose musquée.

Dans encore d'autres conditions préférentielles de mise en oeuvre de l'invention, les compositions ci-dessus renferment en outre de l'huile de centella asiatica.

Dans toujours d'autres conditions préférentielles de mise en oeuvre de l'invention, les compositions ci-dessus renferment en outre de la cire d'abeille et un ou plusieurs extraits tels que des huiles, de pépin de raisin, de germe de blé, d'amandes douces, de lin, de ricin, de symphytum officinalis, de mimosa tenuiflora, d'olive, de carthame, d'onagre, de noir de macadamia, de soja, d'aloès, de romarin et de borreria.

Les compositions selon l'invention renferment de préférence 1 de ces composants supplémentaires, notamment 2, notamment 4, notamment 8 de ceux-ci.

Les différents constituants ci-dessus peuvent entrer dans les compositions selon l'invention dans des proportions variables.

Toutefois, dans des conditions préférentielles de mise en oeuvre de l'invention, une composition selon l'invention terminée comprend pondérale ment :
- de 5 à 35 % d'amidon de riz,
- de 10 à 60 % d'huile de coco,
- de 3 à 15 % de beurre de karité.

Dans d'autres conditions préférentielles de mise en oeuvre de l'invention, les compositions ci-dessus renferment :
- de 1,5 à 10 % d'huile de bourrache
- de 1,5 à 10 % d'huile d'avocat
- de 1 à 5 % d'huile de jojoba.

Dans encore d'autres conditions préférentielles de mise en oeuvre de l'invention, les compositions ci-dessus renferment :
- de 0,2 à 1,5 % d'huile d'argan
- de 0,2 à 1,5 % de rose musquée
- de 0,2 à 1,5 % de centella asiatica.

Dans toujours d'autres conditions préférentielles de mise en oeuvre de l'invention, les compositions ci-dessus renferment :
- de 11 à 25 % d'amidon de riz,
- de 20 à 50 % d'huile de coco,
- de 4 à 10 % de beurre de karité,
- de 2 à 7 % d'huile de bourrache,
- de 2 à 7 % d'huile d'avocat,
- de 1,5 à 4 % d'huile de jojoba.

Les compositions selon l'invention peuvent être fabriquées selon les méthodes usuelles de la cosmétique.

Dans des conditions préférentielles de réalisation, on mixe à température ambiante l'amidon de riz et le coco notamment sous forme d'huile, éventuellement des extraits optionnels comme l'huile de pépin de raisin, l'huile d'olive ou l'huile de ricin, on chauffe au bain-marie à 100°C sous agitation, parallèlement, on mélange à température ambiante le beurre de karité, l'huile de bourrache, l'huile d'avocat et l'huile de jojoba, on porte au bain-marie à environ 75°C sous agitation de 4-5000 tours par minute environ pendant par exemple une heure, on mélange alors les deux préparations ci-dessus et on porte encore au bain-marie à 65-70°C, sous agitation, de préférence d'environ 3 000 tours par minute. On ajoute alors si désiré de la cire d'abeille fondue au bain-marie. Dans un tel cas, on porte sous agitation de préférence à environ 5 000 tours par minute au bain-marie à 70°C, puis laisse refroidir en conservant l'homogénéisation par une agitation réduite. Puis on réduit l'agitation pour obtenir le produit attendu qui dans ce cas se trouve sous forme de crème.

Dans le cas où l'on veut ajouter des composants optionnels, ceux-ci sont ajoutés avantageusement avant l'addition de la cire d'abeille. On peut ainsi ajouter en particulier de l'huile de rose musquée, de la centella asiatica ou d'autres composés cités ci-dessus. L'huile d'argan, pour sa part, est de préférence utilisée sous forme d'huile essentielle de noix.

Les divers constituants mis en jeu dans la présente invention sont avantageusement utilisés sous forme d'huile essentielle obtenue par extraction à l'aide d'un solvant en général alcoolique. Toutefois l'homme de l'art appréciera que ces constituants peuvent être mis en oeuvre sous forme de différents extraits, de préférence liquide, obtenus notamment par décoction, macération ou infusion.

Les compositions dermo-cosmétologiques objet de la présente invention possèdent de très intéressantes propriétés. Elles sont notamment capable d'apporter à la peau - derme et épiderme - une solution compensatrice à la loi naturelle du vieillissement.

Le vieillissement de la peau correspond, entre autres explications, au détachement de l'épiderme (partie superficielle et externe de la peau), de la partie plus profonde que constitue le derme. Ce détachement, faute de soutien entre les deux tissus, bien souvent accentué par le seul fait de la pesanteur, donne naissance à l'apparition de micro crevasses que l'on appelle rides, ridules ou pattes d'oie. Tout se passe alors comme si un « vide » s'installait entre les deux tissus.

De plus, chaque tissu accumule des cellules vieillies lesquelles se reproduisent à un rythme qui va en se ralentissant L'ensemble des tissus sont alors moins vigoureux et moins jeunes.

Par ailleurs, les cellules étant moins riches en collagène et en élastine, les tissus qui les contiennent deviennent moins souples.

Les multiples agressions auxquelles la peau est soumise se traduisent par une accélération de l'oxydation cellulaire qui devient forte et dévastatrice.

Elle génère alors les éléments nocifs appelés radicaux libres. Ils sont la représentation de la dégradation de l'épithélium par suite de l'oxydation cellulaire.

Une composition dermo-cosmétologique selon la présente invention est capable de nourrir les cellules et les tissus de la peau. De plus, cette séparation et cette oxydation sont retardées, voire enrayées. Egalement, elle aide à la reconstitution des facultés de reproduction normale des tissus. Ceux-ci se restructurent et se re densifient ; agissant ainsi, on comble les espaces intercellulaires ou interstices. Cette reconstruction s'organise grâce au concours de cellules nouvelles et jeunes qui contribuent à redonner élasticité et tonicité aux liaisons intercellulaires et aux tissus. Elles sont également plus à même de se développer et de se multiplier et elles vont logiquement se déployer là où il y a de la place, c'est à dire dans les « vides » interstitiels. On assiste ainsi à un comblement de la ride ou à une atténuation des cicatrices.

Par exemple une composition dermo-cosmétologique selon la présente invention sous forme de crème, produit, plus ou moins rapidement suivant la qualité des peaux traitées, les effets suivants :

En surface, elle apporte une sensation de douceur, de satinage, légèrement poudré et de fraîcheur. Elle hydrate la peau, l'adoucit et donne immédiatement une sensation de « rajeunissement » et de « renaissance ». Elle donne à la peau un effet « éclat » par le développement d'une certaine transparence légèrement brillante.

En surface, elle assure une protection contre les agents corrosifs extérieurs et développe les défenses naturelles de l'épithélium.

Elle concourt à nourrir et à hydrater en profondeur l'épiderme et le derme (kératocytes et mélanocytes).

Elle tonifie et stimule les tissus et développe l'activité cellulaire ; cette « régénérescence » favorise la constitution et augmente la densité d'élastine et de collagène dans les tissus. De ce fait, elle améliore l'effet « tenseur » qui est une conséquence de cette « restructuration ».

En amoindrissant les rides, ridules et pattes d'oie et en limitant la dégradation radiculaire, elle est « anti-rides ».

Elle retarde et contient les effets du vieillissement : c'est un « anti-âge » ou « anti-vieillissement ».

Elle contribue à effacer les vergetures ce qui justifie son utilisation lors de soins en période pré et post natale et, elle intervient favorablement dans l'aide à la cicatrisation post chirurgicale et dans le soutien des tissus brûlés ou blessés.

Elle limite les effets du relâchement des tissus en particulier en « remplissant » les interstices vides et espaces inter cellulaires ; elle concourt au soutien du buste et procure un effet « galbe des seins ».

Assurant une meilleure translation des cellules pelliennes, elle stimule une meilleure gestion interne des mélanocytes et elle évite l'accumulation inesthétique de mélanine sous la peau qu'elle tend à disperser ce qui a pour effet d'effacer les « tâches brunes ».

Elle limite le champ d'apparition des cernes et des poches sombres du contour des yeux en nourrissant l'environnement des muscles peauciers.

Elle revitalise le cuir chevelu et les ongles.

Elle atténue, par lubrification des tissus, les douleurs articulaires et rhumatismales.

Elle participe à la reconstruction des tissus cutanés abîmés.

Elle facilite les cicatrisations chromiques, hypertrophiques, rétractiles et chéloïdes.

Dans sa version dotée de particules solides elle « gomme » les cellules mortes et abîmées et développe un effet « exfoliant » ou de « gommage ».

Elle donne à la peau en même temps qu'un effet de transparence, une apparence de « blanchiment »

La présence de coco présente des propriétés naturelles d'écran solaire.

Ces propriétés sont illustrées ci-après dans la partie expérimentale. Elles justifient l'utilisation des compositions ci-dessus décrites en dermocosmétologie.

Les compositions dermo-cosmétologiques selon la présente invention trouvent leur emploi par exemple dans le traitement cosmétique tant curatif que préventif du vieillissement de la peau, des vergetures, des rides, des cicatrices, des taches d'hyper pigmentation.

Elles trouvent aussi leur emploi dans le traitement de certaines blessures, brûlures, gerçures et coupures.

Elles apportent, de par leur composition, un soutien aux tissus déshydratés ou traumatisés (suites de coups ou meurtrissures) ou malades dans le cas de certains herpès, engelures, escarres, etc. Elles délivrent une aide aux soins apportés dans le cas de couperose, acnés et autres dermatoses. Egalement, par l'apport nutritif auxquelles elles concourent, elles améliorent les traitements de certaines douleurs rhumatismales et articulaires, et enrichissent la structure des phanères tels que cheveux et ongles.

Enfin, d'une manière générale, l'utilisateur ressent une sensation appréciable d'apaisement et de protection cutanée.

La dose usuelle, variable selon le sujet et l'effet recherché, peut être, par exemple, de 1 à 10 cm³ par jour par voie externe sur le visage d'une composition de l'exemple 1, pendant 1 mois, de préférence après lavage du visage.

Ces compositions dermo-cosmétologiques peuvent être, par exemple, crémeuses ou liquides et se présenter sous les formes galéniques couramment utilisées en cosmétologie, comme par exemple les crèmes, baumes, laits ou lotions corporels.; elles sont préparées selon les méthodes usuelles. Le ou les principes actifs peuvent y être incorporés à des excipients habituellement employés dans ces compositions pharmaceutiques, tels que, les véhicules aqueux ou non, les corps gras d'origine animale ou de préférence végétale, les dérivés paraffiniques, les divers agents mouillants, dispersants ou émulsifiants, les conservateurs.

Les compositions selon l'invention peuvent également renfermer des particules solides, afin de conférer un effet « peeling » ou exfoliant

On peut utiliser à cet effet les particules solides habituellement utilisées dans ce but, telles que des billes de polyéthylène, particulièrement de nylon et notamment des particules de nature végétale telles que des poudres de noyaux par exemple d'olive ou de cerise réduits à l'état de microsphères.

Des compositions selon l'invention peuvent renfermer en particulier les adjuvants usuels tels que des conservateurs comme les parabens, des fixateurs naturels anti-oxydants tels que la lécithine de soja ou la vitamine C, etc. Les compositions selon l'invention peuvent renfermer des arômes ou des colorants si désiré.

Les exemples qui suivent illustrent la présente demande.

### Exemple 1 : Crème

### Préparation A :

On mixe au couteau et à température ambiante :
- huile de pépin de raisin : 230 g
- huile d'olive : 15 g
- huile de ricin : 35 g
- amidon de riz1 : 100 g.

Quand l'amidon est bien mêlé, on ajoute :
- huile de coco : 200 g.

On fait chauffer l'ensemble au bain-marie à 100°C pendant 75 minutes, en maintenant un brassage léger à 2 000 tours/minute environ puis filtre à chaud.

### Préparation B :

Parallèlement à la préparation A, dans un autre récipient, on mêle, à froid, les ingrédients et huiles suivants :
- beurre de karité : 40 g
- huile de lin : 30 g
- huile de bourrache : 25 g
- huile d'avocat : 25 g
- huile d'argan : 20 g
- huile d'onagre : 20 g
- huile de jojoba : 15 g
- huile de macadamia : 15 g
- huile de carthame : 15 g
- -huile de soja : 10 g
- huile de germe de blé : 10 g
- -huile d'amande douce : 10 g.

Le mixage de cette deuxième préparation s'effectue en portant au bain marie aux environ de 75°c à 4-5 000 tours/minute pendant une heure puis filtre à chaud.

On mélange alors les deux préparations et on assure un brassage de 2 heures à 65-70°c à 3 000 tours/minute.

A la fin de ce traitement, on ajoute 45 g de cire d'abeille fondue au bain marie.

Dans un premier temps, on brasse à 5 000 tours/minute et 70°C pendant 15 minutes, on laisse refroidir en garantissant l'homogénéisation par un léger brassage à 2 000 tours/minute puis on réduit progressivement la fréquence du brassage et la vitesse pour obtenir 850 g de crème (1 litre).

Quand la crème est rigidifiée, on la ramollit au bain marie pour l'empoter.

### Exempte 2 : Crème

On opère comme à l'exemple 1 mais on ajoute, avant l'introduction de la cire d'abeille mais dans les mêmes conditions, les ingrédients suivants :
- huile de rose musquée : 15 gouttes
- symphytum officinal (consoud grande) : 15 gouttes
- aloes : 15 gouttes
- rosmarinus officinalis (romarin) : 15 gouttes
- mimosa tenuiflora (tepezcohuité) : 15 gouttes
- centella asiatica 15 gouttes
- borreria : 15 gouttes

### Exemple 3 Crème avec grains solides

On mixe au couteau et à température ambiante :
- huile de pépin de raisin: 230 g
- huile d'olive : 15 g
- huile de ricin : 35 g
- amidon de riz : 100 g.

Quand l'amidon est bien mêlé, on ajoute :
- huile de coco : 50 g.
- poudre de coco atomisée : 200 g.

On fait chauffer l'ensemble au bain-marie à 100°C pendant 120 minutes, en maintenant un brassage léger à 2 000 tours/minute environ puis filtre à chaud.

### Préparation B :

Parallèlement à la préparation A, dans un autre récipient, on mêle, à froid, les ingrédients et huiles suivants :
- beurre de karité : 40 g
- huile de lin : 30 g
- huile de bourrache : 25 g
- huile d'avocat : 25 g
- huile d'argan : 20 g
- huile d'onagre : 20 g
- huile de jojoba : 15 g
- huile de macadamia : 15 g
- huile de carthame : 15 g
- -huile de soja : 10 g
- huile de germe de blé : 10 g
- -huile d'amande douce : 10 g.

Le mixage de cette deuxième préparation s'effectue en portant au bain marie à 75°c en agitant à 4-5 000 tours/minute pendant une heure, puis filtre à chaud.

On mélange alors les deux préparations et on assure un brassage de 2 heures à 65-70°c à 3 000 tours/minute.

A la fin de ce traitement, on ajoute 45 g de cire d'abeille fondue au bain marie.

Dans un premier temps, on brasse à 5 000 tours/minute et 70°C pendant 15 minutes, on laisse refroidir en garantissant l'homogénéisation par un léger brassage à 2 000 tours/minute puis on réduit progressivement la fréquence du brassage et la vitesse pour obtenir 900 g de crème exfoliante.

Quand la crème est rigidifiée, on la ramollit au bain marie pour l'empoter.

### Exemples d'application

Cas n° 1 : J. A. R. (30 ans) peau noire (Afrique)
Cas n° 2 : V. M. (46 ans) peau noire (Afrique)
Cas n° 3 : W. R. (61 ans) peau blanche nordique
Cas n° 4 : R. F. (72 ans) peau blanche
Cas n° 5 : J. V. (52 ans) peau blanche
Cas n° 6 : M.G. D. (81 ans) peau blanche
Cas n° 7 : I. R. (28 ans) peau noire (Afrique)
Cas n° 8 : C. F. (30 ans) peau métisse
Cas n° 9 : M. M. (48 ans) peau noire (Antilles)
Cas n° 10 : G. P. (31 ans) peau noire (Afrique)
Cas n° 11 : B. G. (50 ans) peau blanche
Cas n° 12 : O. A. (29 ans) peau noire (Afrique)
Cas n° 13 : O.M. (13 ans) peau noire (Afrique)
Cas n° 14 : Q. M. (13 ans) peau noire (Afrique)
Cas n° 15 : L S. (42 ans) peau noire (Afrique)
Cas n° 16 : N. M. (40 ans) peau noire (Afrique)
Cas n° 17 : C. B. (35 ans) peau jaune (Cambodge)
Cas n° 18 : M.C. M. (45 ans) peau rousse.

Les différents types de peau ont été sélectionnés pour couvrir une variété intéressante de situations, en particulier, au niveau des différences de comportement (sécheresse) de la surface externe de la peau.

Il est également important de noter que les personnes sondées ne sont pas de même sexe et n'avaient pas les mêmes pratiques et les mêmes habitudes de traitement de leur peau (méthode, régularité et procédé différent).

Aucune réaction cutanée ni aucune allergie n'ont été signalées.

### Cas n° 1 : Test constant sur 1996 - 97 - 98 et 99.

Le sujet a procédé comme suit : il a appliqué 2 fois par jour 1 noisette de ma formule de l'exemple 1 pendant 3 mois environ.

A noter qu'il présente une cicatrice chéloïde inflammatoire résultant d'une opération de l'appendicite effectuée en 1996 qui a bien évolué suite à 2 applications quotidiennes. Le bourrelet disgracieux est, en partie, résorbé ; les tensions, parfois douloureuses, se sont atténuées.

Par ailleurs un enrichissement de la douceur de la peau, très sujette à une forte sensibilité à la dessiccation, a pu être observée sur le visage, les mains, les avant bras et les jambes.

Les vertus du produit ont pu être observées sur l'entretien avantageux de la poitrine. L'effet tenseur a pu y être démontré.

### Cas n° 2 :

Test après une 1^{ère} naissance tardive (à plus de 40 ans) avec apparition de vergetures (97)

Le sujet a procédé comme suit : il a appliqué 4 noisettes le soir sur le ventre et 1 bonne noisette 2 fois par jour sur la poitrine de l'exemple 1 pendant 2 mois.

Le traitement a permis, sous 4 mois d'application, d'obtenir une forte réduction des vergetures et un excellent retour de l'élasticité abdominale.

Après la montée de lait et les tétées, le retour des seins à une situation normale a été accélérée par l'apposition quotidienne du produit testé.

### Cas n° 3 :

Le sujet a procédé comme suit : il a appliqué 2 noisettes le soir avant le coucher et 1 le matin de l'exemple 3 sur une période de 1 mois renouvelée au bout de 3 mois.

Le produit a été testé après une intervention de chirurgie esthétique au visage. En 2 application quotidiennes, une nette amélioration de la situation des rides a pu être observée et parallèlement les traces cicatricielles ont été gommées sur 3 mois. La restructuration du tissu a été rapide.

Parallèlement, un « collier » de rides a pu fortement être résorbé.

### Cas n° 4 :

Le sujet a procédé comme suit : il a appliqué 2 noisettes matin et soir de l'exemple 3 pendant 6 mois. sur des mains abîmées et plus accessoirement sur un visage marqué par l'âge

Il s'agissait surtout d'apporter un sentiment de bien être et de douceur de l'épiderme. L'intéressé a été très positif sur ces 2 points ; il a convenu de l'amélioration de la texture de la peau de ses mains et de l'atténuation des rides du visage.

### Cas n° 5 :

Le sujet a procédé comme suit : il a appliqué 2 noisettes le matin et 1 le soir a été institué de l'exemple 3.pendant 2 mois.

Il présentait des séquelles importantes résultants d'une situation un peu négligée et des suites d'une exposition forte au soleil sans précautions suffisantes. Les résultats, visibles au bout d'une dizaine de jours, ont été probants sur le comblement des rides en particulier sur la lèvre supérieure et sur le contour des yeux. Egalement, un effet bénéfique a été relevé sur les cheveux et un avantage appréciable sur le cou et le décolleté.

### Cas n° 6 :

Le sujet a procédé comme suit : il a appliqué 1,5 noisettes le matin et 2 le soir de l'exemple 1 pendant 3 mois sur la cicatrice

Ce sujet présentait, à la fois, une usure normale liée au temps, mais aussi, en raison d'une importante intervention chirurgicale au niveau de la cuisse gauche, les conséquences d'une large cicatrice de plus de 25 cm (fracture du fémur).

La cicatrice a fort bien réagi. Elle a réduit tant en longueur qu'en épaisseur.

Par ailleurs, au visage, malgré des applications intermittentes du produit testé, un processus d'amélioration a pu être relevé.

Les tâches des mains ont régressé en intensité et en nombre.

### Cas n° 7 :

Le sujet a procédé comme suit : Elle a appliqué une demi noisette le matin et une le soir de l'exemple 3 pendant 2 mois.

Cette jeune femme noire est constamment affectée par une forte séborrhée et une variété d'acné difficile à résorber sur les joues. Elle a testé avec efficacité le produit et a relevé une amélioration de la tension externe de sa peau. Néanmoins, le traitement un peu négligent n'a pas permis de déceler une vraie solution pour le trouble relevé (acné). Elle a déclaré avoir été très satisfaite du résultat obtenu sur ses cheveux, en général assez peu soignés.

### Cas n° 8 :

Le sujet a procédé comme suit : il a appliqué 1 noisette le soir avant le coucher de l'exemple 1 pendant 6 semaines.

Les caractéristiques de la peau de ce témoin sont celles d'une couleur intermédiaire. Assez grasse naturellement, elle ne présente pas, sauf en ce qui concerne l'expression, de rides clairement définies.

Le sujet a reconnu des résultats satisfaisants et un sentiment de plaisir procuré par réactivité immédiate de la peau en terme de satinage et de douceur.

### Cas n° 9 :

Le sujet a procédé comme suit : il a appliqué 1 noisette matin et soir de l'exemple 3 pendant 1 mois sur les mains assez exposées aux détergents et travaux manuels divers..

Cette femme a jugé très positive l'utilisation du produit malgré un usage limité aux mains.

### Cas n°10:

Le sujet a procédé comme suit : il a appliqué 1 noisette par jour sur les mains de l'exemple 1 pendant 1 mois, et 1 noisette le soir sur les cicatrices pendant 2 mois sur le visage

Concernant un sujet sceptique et réservé, ce cas est intéressant car la peau concernée avait précédemment été blessée par des produits aux qualités douteuses. Les vertus du produit testé sur les mains puis le visage ont été appréciées.

Ultérieurement une application régulière 2 fois par jour sur des cicatrices récentes provenant d'une opération chirurgicale à la poitrine ont apporté une très sensible bonification reconnue et appréciée. L'atténuation des boursouflures et la régénérescence des tissus ont permis une régression sensible de l'aspect visuel de la plaie et une diminution des volumes et dimensions des cicatrices.

### Cas n° 11 :

Le sujet a procédé comme suit : il a appliqué 1 noisette le matin et soir de l'exemple 3 pendant 6 semaines.

Cette femme très habituée à assurer un entretien régulier de sa peau a eu plaisir à tester le produit et s'est dite prête à l'utiliser couramment. Hors des considérations de texture et de gras, elle a apprécié le caractère nourrissant rapide et efficace de la peau.

### Cas n° 12 :

Le sujet a procédé comme suit : il a appliqué 1 noisette le matin et le soir de l'exemple 3 pendant 6 semaines

Cette jeune femme est très attentive à sa beauté. Elle a jugé que le produit lui convenait et qu'elle pouvait en tirer profit pour un entretien quotidien.

### Cas n° 13 :

Le sujet a procédé comme suit : il a appliqué 1 noisette de produit de l'exemple 1 le soir chaque jour pendant 1 mois.

Cette adolescente vit depuis peu en France. Sa puberté a apporté quelques modifications superficielles de l'hydratation épidermique qu'elle a bien compensé avec le produit testé.

### Cas n° 14 :

Le sujet a procédé comme suit : il a appliqué 1 noisette de produit de l'exemple 1 le soir chaque jour pendant 1 mois.

Il s'agit de la soeur jumelle du cas précédent. Le sujet présente une situation strictement identique mais avec une accentuation liée à un mode de vie africain.

### Cas n°15 :

Le sujet a procédé comme suit : il a appliqué 1 noisette matin et soir pendant 2 mois de la crème de l'exemple 3, sur le visage et sur les mains.

Cette femme est très sensible aux notions de soins et d'esthétique corporelle. Elle s'est dite très intéressée pour poursuivre le test plus durablement.

### Cas n° 16 :

Le sujet a procédé comme suit : il a appliqué 1 noisette matin et soir pendant 15 jours de la crème de l'exemple 3.

Cette femme métisse est sensible au soleil et aux effets du vent. Elle a découvert avec satisfaction le côté écran et protecteur de la crème. Elle l'a jugé nourrissante, apaisante et adoucissante.

### Cas n° 17 :

Le sujet a procédé comme suit : il a appliqué une demi noisette le matin et une noisette le soir de la crème de l'exemple 1 pendant 1 mois.

Cette peau de couleur jaune est perturbée par une vie professionnelle assez exposée aux agents polluants : vapeurs, fortes chaleurs, passage fréquent dans des eaux de nettoyage. Un sentiment de confort et d'amélioration a été observé.

### Cas n° 18 :

Le sujet a procédé comme suit : il a appliqué 1 noisette le matin et une demi noisette le soir de la crème de l'exemple 1 pendant 2 mois

Ce sujet a une peau dite rouge, sensible aux rayons ultra violets et qui est vite marquée par un excès d'ensoleillement. L'aspect écran a pu être mis en évidence et une nette réparation des rides a été observée.

## Revendications

1. Une composition dermo-cosmétologique **caractérisée en ce qu'**elle comprend de l'amidon de riz, du coco sous forme de poudre de pulpe, de beurre ou d'huile, du beurre de karité, de l'huile de bourrache, de l'huile d'avocat, de l'huile de jojoba.

2. Une composition dermo-cosmétologique selon la revendication 1, **caractérisée en ce qu'**elle comprend en outre de l'huile de noix d'argan.

3. Une composition dertno-cosmétologique selon la revendication 1 ou 2, **caractérisée en ce qu'**elle comprend en outre de l'huile de rose musquée.

4. Une composition dermo-cosmétologique selon l'une des revendications 1 à 3, **caractérisée en ce qu'**elle comprend en outre de l'huile de centella asiatica.

5. Une composition dermo-cosmétologique selon l'une des revendications 1 à 4, **caractérisée en ce qu'**elle renferme pondérale ment :
- de 5 à 35 % d'amidon de riz,
- de 10 à 60 % d'huile de coco,
- de 3 à 15 % de beurre de karité.

6. Une composition demo-cosmétologique selon la revendication 5, **caractérisée en ce qu'**elle renferme pondérale ment :
- de 1,5 à 10 % d'huile de bourrache,
- de 1,5 à 10 % d'huile d'avocat,
- de 1 à 5 % d'huile de jojoba.

7. Une composition dermo-cosmétologique selon la revendication 5 ou 6, **caractérisée en ce qu'**elle renferme pondérale ment :
- de 0,2 à 1,5 % d'huile d'argan,
- de 0,2 à 1,5 % de rose musquée,
- de 0,2 à 1,5 % de centella asiatica.

8. Une composition dermo-cosmétologique selon l'une des revendications 1 à 7, **caractérisée en ce qu'**elle comprend en outre de la cire d'abeille et des extraits tels que des huiles de pépin de raisin, de germe de blé, d'amande douce, de lin, de ricin, de symphytum officinalis, de mimosa tenuiflora, d'olive, de carthame, d'onagre, de noir de macadamia, de soja, d'aloès, de romarin et de borreria.

9. Une composition dermo-cosmétologique selon l'une des revendications 1 à 8, **caractérisée en ce qu'**elle est formulée sous forme de crème ou de lait.

10. Une composition dermo-cosmétologique **caractérisé en ce qu'**elle renferme :
- de 11 à 25 % d'amidon de riz,
- de 20 à 50 % d'huile de coco,
- de 4 à 11 % de beurre de karité,
- de 2 à 7 % d'huile de bourrache,
- de 2 à 7 % d'huile d'avocat,
- de 1,5 à 4 % d'huile de jojoba.

## Patentansprüche

1. Dermokosmetologische Zusammensetzung, **dadurch gekennzeichnet, dass** sie Reisstärke, Kokos in Form von Pulpenpulver, Butter oder Öl, Karitebutter, Borretschöl, Avocadoöl, Jojobaöl umfasst.

2. Dermokosmetologische Zusammensetzung nach Anspruch 1, **dadurch gekennzeichnet, dass** sie weiters Argannussöl umfasst.

3. Dermokosmetologische Zusammensetzung nach Anspruch 1 oder 2, **dadurch gekennzeichnet, dass** sie weiters Moschusrosenöl umfasst.

4. Dermokosmetologische Zusammensetzung nach einem der Ansprüche 1 bis 3, **dadurch gekennzeichnet, dass** sie weiters Centella-Asiatica-Öl umfasst.

5. Dermokosmetologische Zusammensetzung nach einem der Ansprüche 1 bis 4, **dadurch gekennzeichnet, dass** sie gewichtsmäßig enthält:
- 5 bis 35 % Reisstärke,
- 10 bis 60 % Kokosöl,
- 3 bis 15 % Karitebutter.

6. Dermokosmetologische Zusammensetzung nach Anspruch 5, **dadurch gekennzeichnet, dass** sie gewichtsmäßig enthält:
- 1,5 bis 10 % Borretschöl,
- 1,5 bis 10 % Avocadoöl,
- 1 bis 5 % Jojobaöl.

7. Dermokosmetologische Zusammensetzung nach Anspruch 5 oder 6, **dadurch gekennzeichnet, dass** sie gewichtsmäßig enthält:
- 0,2 bis 1,5 % Arganöl,
- 0,2 bis 1,5 % Moschusrosenöl,
- 0,2 bis 1,5 % Centella Asiatica.

8. Dermokosmetologische Zusammensetzung nach einem der Ansprüche 1 bis 7, **dadurch gekennzeichnet, dass** sie weiters Bienenwachs und Extrakte wie Traubenkernöl, Weizenkeimöl, Süßmandelöl, Leinöl, Rizinusöl, Symphytum-Officinalis-Öl, Mimosa-Tenuiflora-Öl, Olivenöl, Carthame-Öl, Nachtkerzenöl, Makadamianussöl, Sojaöl, Aloeöl, Rosmarinöl und Borreriaöl umfasst.

9. Dermokosmetologische Zusammensetzung nach einem der Ansprüche 1 bis 8, **dadurch gekennzeichnet, dass** sie in Form einer Creme oder Milch formuliert ist.

10. Dermokosmetologische Zusammensetzung, **dadurch gekennzeichnet, dass** sie enthält:
- 11 bis 25 % Reisstärke,
- 20 bis 50 % Kokosöl,
- 4 bis 11 % Karitebutter,
- 2 bis 7 % Borretschöl,
- 2 bis 7 % Avocadoöl,
- 1,5 bis 4 % Jojobaöl.

## Claims

1. A cosmetic composition for skin care, **characterized in that** it contains rice starch, coconut in the form of powdered pulp, butter or oil, shea butter, borage oil, avocado oil, jojoba oil.

2. A cosmetic composition for skin care according to claim 1, **characterized in that** it additionally contains argan oil.

3. A cosmetic composition for skin care according to claim 1 or 2, **characterized in that** it additionally contains musk rose oil.

4. A cosmetic composition for skin care according to one of the claims 1 to 3, **characterized in that** it additionally contains oil of centella asiatica.

5. A cosmetic composition for skin care according to one of claims 1 to 4, **characterized in that** it contains by weight:
- from 5 to 35 % rice starch,
- from 10 to 60 % coconut oil,
- from 3 to 15 % shea butter.

6. A cosmetic composition for skin care according to claim 5, **characterized in that** it contains by weight:
- from 1.5 to 10 % borage oil,
- from 1.5 to 10 % avocado oil,
- from 1 to 5 % jojoba oil.

7. A cosmetic composition for skin care according to claim 5 or 6, **characterized in that** it contains by weight:
- from 0.2 to 1.5 % argan oil,
- from 0.2 to 1.5 % musk rose,
- from 0.2 to 1.5 % centella asiatica.

8. A cosmetic composition for skin care according to one of claims 1 to 7, **characterized in that** it additionally contains beeswax and extracts such as grapeseed oil, wheatgerm oil, sweet almond oil, linseed oil, castor oil, oil of symphytum officinale, oil of mimosa tenuiflora, olive oil, safflower oil, evening primrose oil, macadamia oil, soya oil, aloe oil, oil of rosemary and oil of borreria.

9. A cosmetic composition for skin care according to one of claims 1 to 8, **characterized in that** it is formulated in the form of cream or lotion.

10. A cosmetic composition for skin care, **characterized in that** it contains:
- from 11 to 25 % rice starch,
- from 20 to 50 % coconut oil,
- from 4 to 11 % shea butter,
- from 2 to 7 % borage oil,
- from 2 to 7 % avocado oil,
- from 1.5 to 4 % jojoba oil.
